# EUROPEAN PATENT SPECIFICATION

(11) **EP 1 986 791 B1**
(45) Date of publication and mention of the grant of the patent: **28.10.2015**
(21) Application number: 07763398.0
(22) Date of filing: 07.02.2007
(51) Int. Cl.: B05D 3/08, B05D 1/08, B05D 5/00, A61L 31/16

(54) **DRUG DELIVERY SYSTEM AND METHOD OF MANUFACTURING THEREOF**
ARZNEIMITTELVERABREICHUNGSSYSTEM UND HERSTELLUNGSVERFAHREN DAFÜR
SYSTÈME DE DÉLIVRANCE DE MÉDICAMENTS ET SON PROCÉDÉ DE FABRICATION

(30) Priority: 07.02.2006 US 349483; 17.10.2006 US 550069
(43) Date of publication of application: 05.11.2008
(73) Proprietor: Exogenesis Corporation, Billerica, MA 01821 (US)
(72) Inventor: BLINN, Stephen, M., Wellesley Hills, MA 02481 (US); SVRLUGA, Richard, C., Wellesley Hills, Massachusetts 02481 (US)
(74) Representative: Casey, Lindsay Joseph
(86) International application number: PCT/US2007/061787
(87) International publication number: WO 2007/092894

(56) References cited:
- US-A- 5 763 504
- US-A- 6 143 354
- US-A1- 2002 188 324
- US-A1- 2003 143 315
- US-A1- 2005 043 787
- US-A1- 2005 079 200
- US-A1- 2005 244 453
- US-B1- 6 984 404
- US-B2- 6 676 989
- US-B2- 6 746 773

## Description

### Field of the Invention

This invention relates generally to drug delivery systems such as, for example, medical devices implantable in a mammal (e.g., coronary stents, prostheses, etc.), and more specifically to a method and system for applying and adhering drugs to the surface of medical devices and for controlling the surface characteristics of such drug delivery systems such as, for example, the drug release rate and bio-reactivity, using gas cluster ion beam technology in a manner that permits efficacious release of the drugs from the surface over time.

### Background of the Invention

A coronary stent is an implantable medical device that is used in combination with balloon angioplasty. Balloon angioplasty is a procedure used to treat coronary atherosclerosis. Balloon angioplasty compresses built-up plaque against the walls of the blocked artery by the inflation of a balloon at the tip of a catheter inserted into the artery during the angioplasty procedure. Unfortunately, the body's response to this procedure often includes thrombosis or blood clotting and the formation of scar tissue or other trauma-induced tissue reactions at the treatment site. Statistics show that restenosis or re-narrowing of the artery by scar tissue after balloon angioplasty occurs in up to 35 percent of the treated patients within only six months after these procedures, leading to severe complications in many patients.

To reduce restenosis, cardiologists are now often placing small tubular devices of various forms, such as wire mesh; expandable metal; and non-degradable and biodegradable polymers called a coronary stent at the site of blockage during balloon angioplasty. The goal is to have the stent act as a scaffold to keep the coronary artery open after the removal of the balloon.

However, there are also serious complication associated with the use of coronary stents. Coronary restenotic complications associated with stents occur in 16 to 22 percent of all cases within six months after insertion of the stent and are believed to be caused by many factors acting alone or in combination. These complications could be reduced by several types of drugs introduced locally at the site of stent implantation. Because of the substantial financial costs associated with treating the complications of restenosis, such as catheterization, restenting, intensive care, etc., a reduction in restenosis rates would save money and reduce patient suffering.

Numerous studies suggest that the current popular designs of coronary stents are functionally equivalent. Although the use of coronary stents is growing, the benefits of their use remain controversial in certain clinical situations or indications due to their potential complications. It is widely held that during the process of expanding the stent, damage occurs to the endothelial lining of the blood vessel triggering a healing response that re-occludes the artery. To help combat that phenomenon, drug-coated stents are being introduced to the market to help control the abnormal cell growth associated with this healing response. These drugs are typically mixed with a liquid polymer and applied to the stent surface. When implanted, the drug elutes out of the polymer in time, releasing the medicine into the surrounding tissue. There remain a number of problems associated with this technology. Because the stent is expanded at the diseased site, the polymeric material has a tendency to crack and sometimes delaminate from the stent surface. These polymer flakes can travel throughout the cardio-vascular system and cause significant damage. There is some evidence to suggest that the polymers themselves cause a toxic reaction in the body. Additionally, because of the thickness of the coating necessary to carry the required amount of medicine, the stents can become somewhat rigid making expansion difficult. In other prior art stents, the wire mesh of the stent itself is impregnated with one or more drugs through processes such as high pressure loading, spraying, and dipping. However, loading, spraying and dipping do not satisfactorily adhere the drug to the stent surface and therefore, in many instances, do not yield the optimal, time-release dosage of the drugs delivered to the surrounding tissue. The polymer coating can include several layers such as the above drug containing layer as well as a drug free encapsulating layer, which can help to reduce the initial drug release amount caused by initial exposure to liquids when the device is first implanted. A further base coating of polymer located beneath the drug bearing layer is also known. One example of this arrangement used on stainless steel stents includes a base layer of Paralene C. and a drug/polymer mixture including polyethylene-co-vinyl acetate (PEVA) and poly n-butyl methacrylate (PBMA) in a two to one ratio, along with an non-drug impregnated top layer of the same mixture of PEVA and PBMA. The drug used is Sirolimus, a relatively new immunosuppressant drug also known as Rapamycin. Several other drug/polymer combinations exist from several manufactures.

In view of this new approach to in situ drug delivery, it is desirable to have greater control over the drug release rate from the implantable device as well as control over other surface characteristics of the drug delivery medium.

It is therefore an object of this invention to provide a means of applying and adhering drugs to medical devices using gas cluster ion beam technology.

It is a further object of this invention to apply drugs to medical stents by gas cluster ion beams to decrease the complication of restenosis and thrombosis.

it is a further object of this invention to provide a means for controlling surface characteristics of a drug eluting material using gas cluster ion beam technology.

It is a further object of this invention to transform the surfaces of medical devices into drug delivery systems by applying and adhering drugs to the surfaces with gas cluster ion beams so as to facilitate a timed release of the drug(s) from the surfaces.

It is a further object of this invention to improve the functional characteristics of known in said to drug release mechanisms using gas cluster ion beam technology.

The objects set forth above as well as further and other objects and advantages of the present invention are achieved by the invention described herein below.

The present invention is directed to the use of gas cluster ion-beam (GCIB) surface modification to implant, apply, or adhere various drug molecules directly into or onto the surface of a stent or other medical device, thereby eliminating the need for a polymer or any other binding agent and transforming the medical device surface into a drug delivery system. This will prevent the problem of toxicity and the damage caused by transportation of delaminated polymeric material throughout the body. Unlike the prior art stents described above that load the stent material itself, the present invention provides the United States Patent specification no US-A-2002/188324 discloses medical devices implanted or otherwise used in a mammal which are made less prone to trigger adverse reactions by use of gas cluster ion-beam (GCIB) surface modification and/or monomer ion beam surface modification to implant, apply, or adhere various drug molecules directly into or onto the surface of medical devices. This is accomplished without the need for a polymer or any other binding agent to retain the drug.

### Summary of the Invention

According to an aspect of the present invention, there is provided a method of producing a drug delivery system as specified in claim 1. According to another aspect of the present invention, there is provided a drug delivery system as specified in claim 10. ability to adhere for time-release an optimal dosage of the drug or drugs.

The application of the drug(s) is achieved through the use of GCIB technology. The application of the drug(s) is accomplished by several methods:
The surface of the medical device, which may be composed, for example, of a polymer, metal or any other material, is optionally first processed using a GCIB which will remove any contaminants and oxide layers from the surface rendering the surface electrically active and creating dangling bonds. The desired drug will then be deposited upon the active surface and will bond with the dangling bonds.

A second method for producing a drug delivery system involves depositing a layer of one or more drug substances onto at least one surface region of a medical device (which may or may not have been pre-processed with a GCIB) in liquid, powder or other form, perhaps through sublimating the drug, and then impacting the deposited drug layer with an energetic GCIB so as to form an adhered drug layer. The GCIB dose creates a carbonized drug matrix including a plurality of interstices through which non-carbonized drug will diffuse or elute over time. If the deposited drug layer is suitably thin, some GCIB clusters may penetrate through the deposited drug layer and reach the surface of the medical device, such that the adhered drug layer may include some portion of the deposited drug molecules implanted sub-surface in the form of a mechanical bond. If the deposited drug layer has a thickness above a threshold thickness (for a particular GCIB dose), however, the carbonized drug matrix will not be "stitched" to the surface of the medical device. Rather, the carbonized matrix will be formed over the remaining non-carbonized, mobile volume of the deposited drug. In one stent embodiment, for example, a ring-like, carbonized drug matrix is formed concentrically about a layer of non-carbonized, deposited drug which, in turn, is disposed about the stent on the stent surface, with little to no portion of the carbonized matrix directly stitched to the stent surface.

In multi-layered embodiments of the invention, subsequent drug layers may be comprised of identical, similar or distinct drug substances. Additionally, identical, similar or different drug deposition techniques than those used to deposit preceding layers may be employed. Controlled variations in the GCIB characteristics and dosing delivered to different layers (and between spatially distinct regions of a single layer) may also be employed. Substantially similar GCIB doses delivered to substantially similar drug substances will result in similar drug elution profiles, while different doses can achieve distinct inter-layer elution profiles. Judicious selection of drug substance(s), and control over the deposition technique and GCIB dosing permits formation of a drug delivery system comprised of multiple, adhered drug layers each having similar or differing drug elution profiles which, in preferred embodiments of the invention, cooperate to achieve at least one overall drug elution profile. For example, the elution profiles of individual layers may be designed such that, as drug is diffused from the outermost adhered drug layer, it is replenished by drug(s) eluting from lower adhered layers.

A number of techniques may be employed to deposit the drug substance(s) onto the medical device surface, or one or more spatially distinct regions thereof. If the drug is to be deposited in liquid form, techniques such as dipping, spraying, vapor phase deposition, and ultrasonic atomization may be utilized. Alternatively, if the drug is in powder for, it may be electrostatically deposited onto the medical device surface or deposited by sublimation, and then GCIB irradiated in the same manner described above.

Any of the methods described may optionally include an irradiation step prior to drug deposition to obtain a smoother surface, which will help reduce non-uniform thickness in the adhered drug layer(s).

The application of drugs via GCIB surface modification such as described above will reduce complications, lead to genuine cost savings and an improvement in patient quality of life, and overcome prior problems of thrombosis and restenosis. Preferred therapeutic agents for delivery in the drug delivery systems of the present invention include anti-coagulants, antibiotics, immunosuppressant agents, vasodilators, anti-prolifics, anti-thrombotic substances, anti-platelet substances, cholesterol reducing agents, anti-tumor medications and combinations thereof.

In one embodiment, a drug delivery system, comprises a member including a combination of a drug substance and a polymer or other material, and an encapsulating layer formed in an outer surface of the member by gas cluster ion beam irradiation of the outer surface of the member, which encapsulating layer is adapted to determine a release rate for the drug from the member.

The encapsulating layer may include a plurality of openings located at an outer surface of the encapsulating layer and adapted to permit amounts of the drug substance to be released from the member at a rate determined by the encapsulating layer. The encapsulating layer may include a carbonized or densified matrix. The encapsulating layer may be adapted to improve a measure of biocompatibility of the member.

The member may be located on a surface of a medical device. The drug substance may be selected from the group consisting of anti-coagulants, antibiotics, anti-tumor substances, immune-suppressing agents, vasodilators, anti-prolifics, anti-thrombotic substances, anti-platelet substances, cholesterol reducing agents and combinations thereof.

A medical device may include the drug delivery system described above.

In another embodiment, a drug delivery system comprises a cohesive mixture including a combination of a drug substance and a polymer or other material, and a carbonized or densified matrix formed on an outer surface of the cohesive mixture, which carbonized or densified matrix is adapted to determine a release rate for the drug substance from the cohesive mixture.

In yet another embodiment, a method for producing a drug delivery system, comprises the steps of providing a member including a combination of a drug substance and a polymer or other material, and irradiating an outer surface of the member with a gas cluster ion beam to determine a release rate for the drug substance from the member.

The step of providing a member may include forming a cohesive mixture of the drug substance and the polymer or other material on a surface of a medical device. The step of irradiating may include forming an encapsulating layer on at least an external surface of the member, which encapsulating layer is adapted to control release of the drug substance from the member. The encapsulating layer may include a plurality of openings at an outer surface of the encapsulating layer so as to permit portions of the drug substance to be released from the member at a rate determined by the encapsulating layer. The encapsulating layer may include a carbonized or densified matrix.

The step of providing a member may include the steps of providing a polymer element and adhering a drug substance to an outer surface of the polymer element. The step of providing a polymer element may include the step of irradiating the outer surface of the polymer element with a gas cluster ion beam prior to the step of adhering. The step of irradiating may be adapted to lower in situ chemical reactivity of the external surface of the cohesive mixture. The drug substance may be selected from the group consisting of anti-coagulants, antibiotics, anti-tumor substances, immune-suppressing agents, vasodilators, anti-prolifics, anti-thrombotic substances, anti-platelet substances, cholesterol reducing agents and combinations thereof.

### Brief Description of the Drawings

For a better understanding of the present invention, together with other and further objects thereof, reference is made to the accompanying drawings, wherein:
FIG. 1 is a schematic view of a gas cluster ion beam processing system used for practicing the method of the present invention;
FIG. 2 is an exploded view of a portion of the gas cluster ion beam processing system of FIG. 1 showing the workpiece holder;
FIG. 3 is an atomic force microscope image showing the surface of a coronary stent before GCIB processing;
FIG. 4 is an atomic force microscope image showing the surface of a coronary stent after GCIB processing;
FIGS. 5A-5H are illustrations of a surface region of a medical device at various stages of drug delivery system formation in accordance with an embodiment of the present invention;
FIGS. 6A-6B are illustrations of alternative drug delivery structure embodiments in accordance with the present invention;
FIG. 7A is a graph showing the release rate of fluorescence over time;
FIG. 7B is a graph showing the cumulative release rate of fluorescence over time;
FIG. 8 is a graph showing comparative drug elution rate test results for a conventional drug-coated stent and a stent processed in accordance with the present invention.
FIG. 9 is a cross section of a drug delivery system prior to processing in accordance with another embodiment of the present invention; and
FIG. 10 is a cross section of the drug delivery system of FIG. 9 shown during gas cluster ion beam processing performed in accordance with the present invention;

### Detailed Description of the Drawings

Beams of energetic ions, electrically charged atoms or molecules accelerated through high voltages under vacuum, are widely utilized to form semiconductor device junctions, to smooth surfaces by sputtering, and to enhance the properties of semiconductor thin films. In the present invention, these same beams of energetic ions are utilized for the applying and adhering drugs to a surface and for affecting surface characteristics of drug eluting medical devices, such as, for example, coronary stents, thereby converting the surface into a drug delivery system with enhanced drug delivery properties and bio-compatibility.

In the preferred embodiment of the present invention, gas cluster ion beam GCIB processing is utilized. Gas cluster ions are formed from large numbers of weakly bound atoms or molecules sharing common electrical charges and accelerated together through high voltages to have high total energies. Cluster ions disintegrate upon impact and the total energy of the cluster is shared among the constituent atoms. Because of this energy sharing, the atoms are individually much less energetic than the case of conventional ions or ions not clustered together and, as a result, the atoms penetrate to much shorter depths. Surface sputtering effects are orders of magnitude stronger than corresponding effects produced by conventional ions, thereby making important microscale surface effects possible that are not possible in any other way.

The concept of GCIB processing has only emerged over the past decade. Using a GCIB for dry etching, cleaning, and smoothing of materials is known in the art and has been described, for example, by Deguchi, et al. in U.S. Pat. No. 5,814,194, "Substrate Surface Treatment Method", 1998. Because ionized clusters containing on the order of thousands of gas atoms or molecules may be formed and accelerated to modest energies on the order of a few thousands of electron volts, individual atoms or molecules in the clusters may each only have an average energy on the order of a few electron volts. It is known from the teachings of Yamada in, for example, U.S. Pat. No. 5,459,326, that such individual atoms are not energetic enough to significantly penetrate a surface to cause the residual sub-surface damage typically associated with plasma polishing. Nevertheless, the clusters themselves are sufficiently energetic (some thousands of electron volts) to effectively etch, smooth, or clean hard surfaces.

Because the energies of individual atoms within a gas cluster ion are very small, typically a few eV, the atoms penetrate through only a few atomic layers, at most, of a target surface during impact. This shallow penetration of the impacting atoms means all of the energy carried by the entire cluster ion is consequently dissipated in an extremely small volume in the top surface layer during a period on the order of 10⁻¹² seconds (i.e. one picosecond). This is different from the case of ion implantation which is normally done with conventional monomer ions and where the intent is to penetrate into the material, sometimes penetrating several thousand angstroms, to produce changes in the surface properties of the material. Because of the high total energy of the cluster ion and extremely small interaction volume, the deposited energy density at the impact site is far greater than in the case of bombardment by conventional monomer ions.

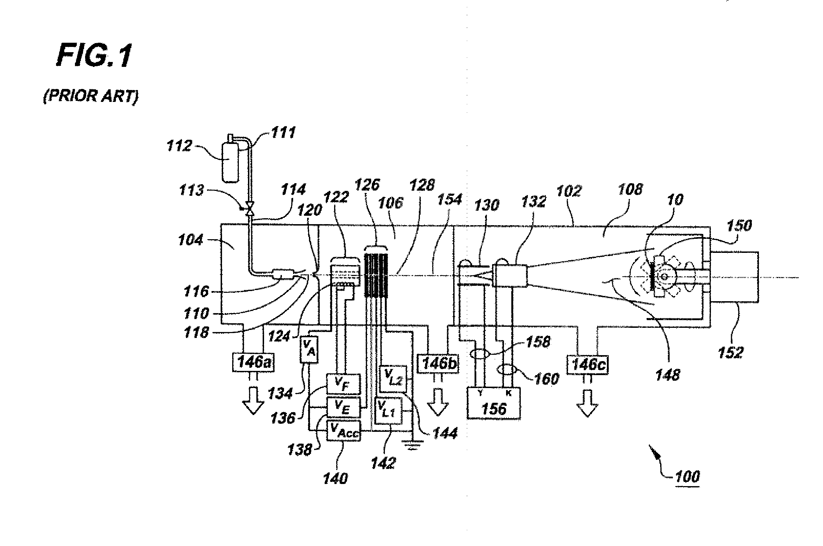

Reference is now made to FIG. 1 of the drawings which shows the GCIB processor 100 of this invention utilized for applying or adhering drugs to the surface of a medical device such as, for example, coronary stent 10. Although not limited to the specific components described herein, the processor 100 is made up of a vacuum vessel 102 which is divided into three communicating chambers, a source chamber 104, an ionization/acceleration chamber 106, and a processing chamber 108 which includes therein a uniquely designed workpiece holder 150 capable of positioning the medical device for uniform GCIB bombardment and drug application by a gas cluster ion beam.

During the processing method of this invention, the three chambers are evacuated to suitable operating pressures by vacuum pumping systems 146a, 146b, and 146c, respectively. A condensable source gas 112 (for example argon or N₂) stored in a cylinder 111 is admitted under pressure through gas metering valve 113 and gas feed tube 114 into stagnation chamber 116 and is ejected into the substantially lower pressure vacuum through a properly shaped nozzle 110, resulting in a supersonic gas jet 118. Cooling, which results from the expansion in the jet, causes a portion of the gas jet 118 to condense into clusters, each consisting of from several to several thousand weakly bound atoms or molecules. A gas skimmer aperture 120 partially separates the gas molecules that have not condensed into a cluster jet from the cluster jet so as to minimize pressure in the downstream regions where such higher pressures would be detrimental (e.g., ionizer 122, high voltage electrodes 126, and process chamber 108). Suitable condensable source gases 112 include, but are not necessarily limited to argon, nitrogen, carbon dioxide, oxygen.

After the supersonic gas jet 118 containing gas clusters has been formed, the clusters are ionized in an ionizer 122. The ionizer 122 is typically an electron impact ionizer that produces thermo-electrons from one or more incandescent filaments 124 and accelerates and directs the electrons causing them to collide with the gas clusters in the gas jet 118, where the jet passes through the ionizer 122. The electron impact ejects electrons from the clusters, causing a portion the clusters to become positively ionized. A set of suitably biased high voltage electrodes 126 extracts the cluster ions from the ionizer 122, forming a beam, then accelerates the cluster ions to a desired energy (typically from 1 keV to several tens of keV) and focuses them to form a GCIB 128 having an initial trajectory 154. Filament power supply 136 provides voltage V_{F} to heat the ionizer filament 124. Anode power supply 134 provides voltage V_{A} to accelerate thermoelectrons emitted from filament 124 to cause them to bombard the cluster containing gas jet 118 to produce ions. Extraction power supply 138 provides voltages V_{E} to bias a high voltage electrode to extract ions from the ionizing region of ionizer 122 and to form a GCIB 128. Accelerator power supply 140 provides voltage V_{Acc} to bias a high voltage electrode with respect to the ionizer 122 so as to result in a total GCIB acceleration energy equal to V_{Acc} electron volts (eV). One or more lens power supplies (142 and 144, for example) may be provided to bias high voltage electrodes with potentials (V_{L1} and V_{L2} for example) to focus the GCIB 128.

A medical device, such as coronary stent 10, to be processed by the GCIB processor 100 is held on a workpiece holder 150, and disposed in the path of the GCIB 128 for irradiation. The present invention may be utilized with medical devices composed of a variety of materials, such as metal, ceramic, polymer, or combinations thereof. In order for the stent to be uniformly processed using GCIB, the workpiece holder 150 is designed in a manner set forth below to manipulate the stent 10 in a specific way.

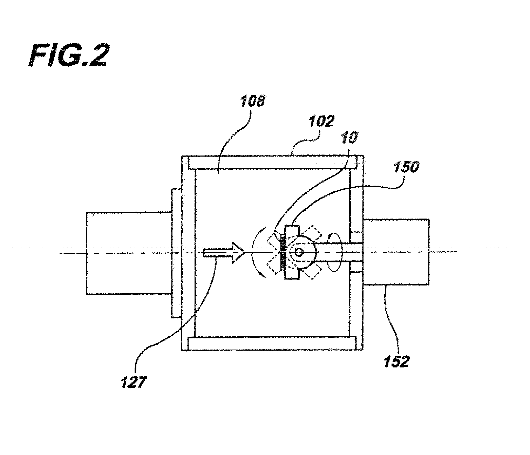

Referring now to FIG. 2 of the drawings, medical device surfaces that are non-planar, such as those of stents, must remain oriented within a specific angle tolerance with respect to the normal beam incidence to obtain paramount effect to the stent surfaces utilizing GCIB. This requires a fixture or workpiece holder 150 with the ability to be fully articulated to orient all non-planar surfaces of stent 10 to be modified within that specific angle tolerance at a constant exposure level for process optimization and uniformity. Any stent 10 containing surfaces that would be exposed to the process beam at angles of greater than +/-15 degrees from normal incidence may require manipulation. More specifically, when applying GCIB to a coronary stent 10, the workpiece holder 150 is rotated and articulated by a mechanism 152 located at the end of the GCIB processor 100. The articulation/rotation mechanism 152 preferably permits 360 degrees of device rotation about longitudinal axis 154 and sufficient device articulation about an axis 156 perpendicular to axis 154 to maintain the stent's surface to within +/-15 degrees from normal beam incidence.

Referring back to FIG. 1, under certain conditions, depending upon the size of the coronary stent 10, a scanning system may be desirable to produce uniform smoothness. Although not necessary for GCIB processing, two pairs of orthogonally oriented electrostatic scan plates 130 and 132 may be utilized to produce a raster or other scanning pattern over an extended processing area. When such beam scanning is performed, a scan generator 156 provides X-axis and Y-axis scanning signal voltages to the pairs of scan plates 130 and 132 through lead pairs 158 and 160 respectively. The scanning signal voltages are commonly triangular waves of different frequencies that cause the GCIB 128 to be converted into a scanned GCIB 148, which scans the entire surface of the stent 10. Additional means for orienting, articulating and/or rotating devices such as stents and orthopedic products are disclosed in U.S. Patent Nos. 6,491,800 to Kirkpatrick, et al., 6,676,989 to Kirkpatrick, et al., and 6,863,786 to Blinn, et al., the contents of each which are hereby incorporated by reference.

When beam scanning over an extended region is not desired, processing is generally confined to a region that is defined by the diameter of the beam. The diameter of the beam at the stent's surface can be set by selecting the voltages (V_{L1} and/or V_{L2}) of one or more lens power supplies (142 and 144 shown for example) to provide the desired beam diameter at the workpiece.

In one processing step related to the present invention, the surface of a medical device is irradiated with a GCIB prior to the deposition of any substance on the surface thereof. This will remove any contaminants and oxide layers from the stent surface rendering the surface electrically active and capable of attracting and bonding drug and polymer molecules that are then introduced to the surface. One or more types of drugs are deposited upon surface through vapor phase deposition or by introducing a liquid form of the drug onto the surface. In some instances, the liquid form of the drug is in solution with a volatile solvent thereby requiring the solvent to be evaporated. As the formed mechanical bonds are broken over time, the drug is slowly released to the site of device implantation.

Studies have suggested that a wide variety of drugs may be useful at the site of contact between the medical device and the in vivo environment. For example, drugs such as anti-coagulants, anti-prolifics, antibiotics, immune-supressing agents, vasodilators, anti-thrombotic substances, anti-platelet substances, and cholesterol reducing agents may reduce instances of restenosis when diffused into the blood vessel wall after insertion of the stent.

In another processing step, GCIB processing is utilized to impact a deposited drug layer (and the surface of the medical device if the deposited drug layer is thin enough to permit gas clusters penetration to the surface) with energetic clusters thus implanting and forming a mechanical bond between the surface and the deposited drug molecules; or to implant the drug molecules of the electrostatically coated or sublimated medicine in powder form to the stent surface in the same manner described above. The impact energy of the gas clusters causes a portion of the deposited drug molecules to form a carbonized drug matrix. As the carbon matrix is formed, the remaining (non-carbonized) drug molecules become embedded within the interstices of the matrix, and/or are encapsulated between the carbon matrix and the medical device surface. Over time, these drug molecules diffuse through the matrix and are released at the contact site between the stent and the blood vessel wall thereby contiguously providing medication to the site.

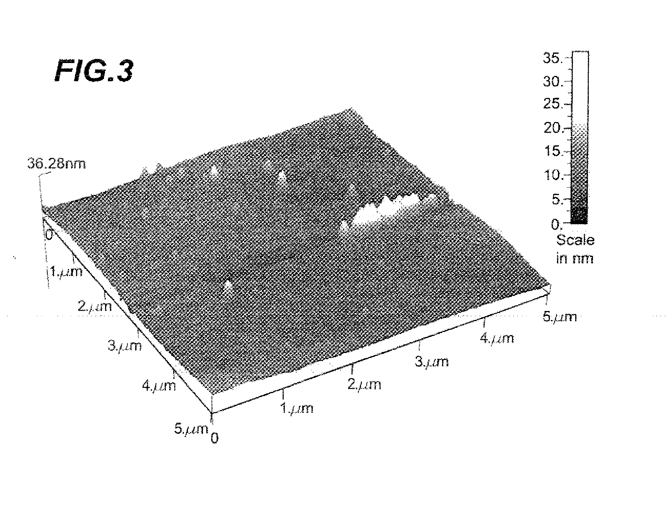

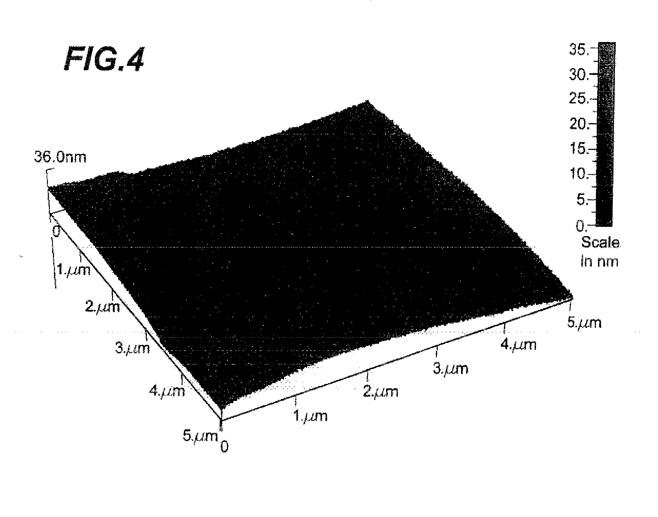

As the atomic force microscope (AFM) images shown in FIGS. 3 and 4 demonstrate, it is possible to dramatically affect the medical device surface utilizing gas cluster ion beam processing. FIG. 3 shows a stent surface before GCIB treatment with gross surface micro-roughness on a strut edge. The surface roughness measured an Rₐ of 113 angstroms and an **R_{RMS}** of 148 angstroms. These irregularities highlight the surface condition at the cellular level where thrombosis begins. FIG. 4 shows the stent surface after GCIB processing where the surface micro-roughness has been eliminated without any measurable physical or structural change to the integrity of the stent itself. The post-GCIB surface roughness measured an Rₐ of 19 angstroms and an R_{RMS} of 25 angstroms. In this manner, GCIB processing also provides the added benefit of smoothing the surface of the medical device. Non-smooth surfaces may snare fibrinogen, platelets, and other matter further promoting stenosis.

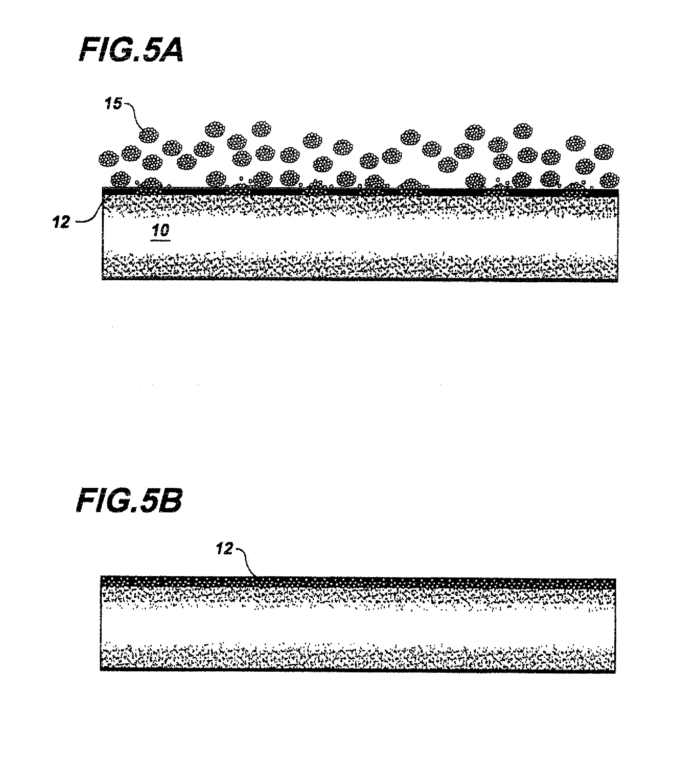

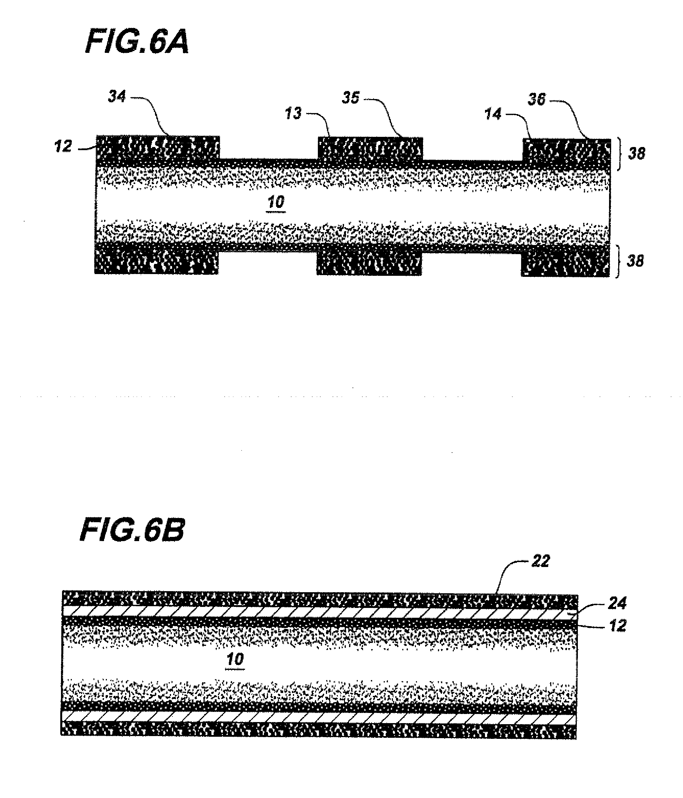

With reference to FIGS. 5A-5F, a method of producing a drug delivery system will now be described. FIG. 5A illustrates a surface region 12 of a medical device such as, for example, stent 10, that has been positioned in a vacuum chamber such that it can be irradiated with gas clusters 15 of a GCIB, as would occur in an optional smoothing process step. FIG. 6A illustrates an exemplary drug delivery structure in accordance with an embodiment of the present invention. Note that the drug delivery structure may cover all or less than the entirety of the exterior surface of stent 10. In the latter case, surface region 12 represents but one of a plurality of spatially distinct surface regions 12-14 of stent 10 upon which the drug delivery system is formed. Each of the distinct surface regions 12-14 may elute the same or similar type of drug, or completely distinct types of drugs. For ease in understanding, the description that follows focuses on the formation of the drug delivery structure at surface region 12 only.

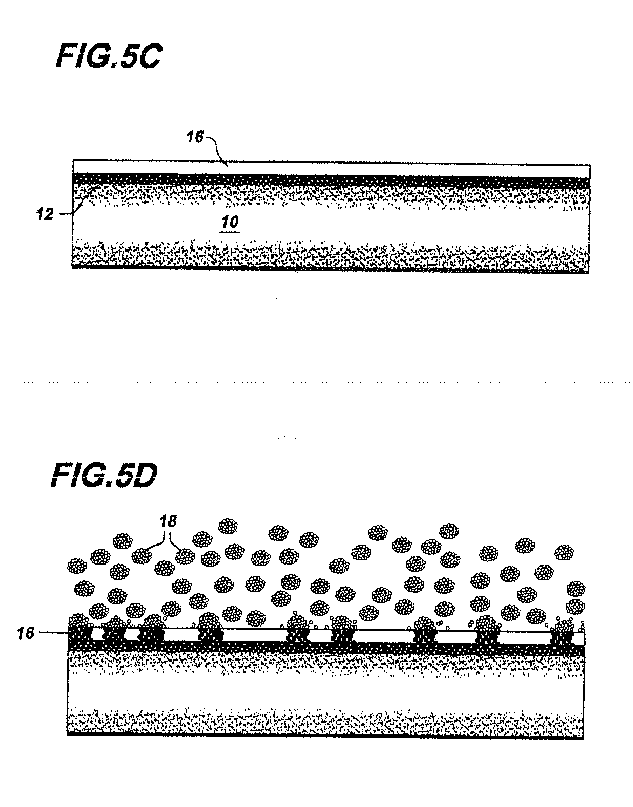

FIG. 5B illustrates surface region 12 as being relatively smooth, following an optional surface preparation step through GCIB irradiation. As described above, such processing removes contaminants and electrically activates the surface region 12. FIG. 5C shows a drug layer 16, which may be deposited by any of the techniques described above, and which preferably has been deposited to have a substantially uniform thickness in the vicinity of region 12. A "deposited drug layer" is used herein to refer to a contiguous drug layer deposited over the entirety of the surface of the medical device, such as deposited drug layer 16, or alternatively may be used in a collective sense to refer to numerous spatially distinct deposits of the same or different therapeutic agents on the surface 12. In either case, the deposited drug layer is GCIB irradiated to form an adhered drug layer on the device surface from which a portion of the deposited agent wil be released over time to a patient's tissue adjacent the medical device.

As the term is used herein, an "adhered drug layer" refers collectively to the post-GCIB irradiated layer comprised of at least one portion of non-carbonized deposited drug substance(s) and at least one carbonized matrix through which the deposited drug substance(s) is released at an expected rate. In embodiments described below, a drug delivery system comprised of multiple, adhered drug layers may subsequently be formed by repeatedly depositing additional layers of a selected drug substance onto a preceding adhered layer and irradiating the additional deposited drug layer with GCIB's. The selection of drug substance types, the method for depositing the drug (including sublimation) onto the medical device surface, and the control over GCIB dosing permits the precise formation of adhered drug layers such that a desired drug release rate, or elution profile, may be achieved in a multi-layered system. Subsequently adhered drug layers wild have very few to no direct bonds between the carbonized matrix associated with the subsequent layer and the surface of the medical device. Rather, such layers will be adhered to preceding drug matrix layers. And in certain embodiments, the carbonized drug matrix of even the first layer will not be bonded, or stitched, to the stent surface.

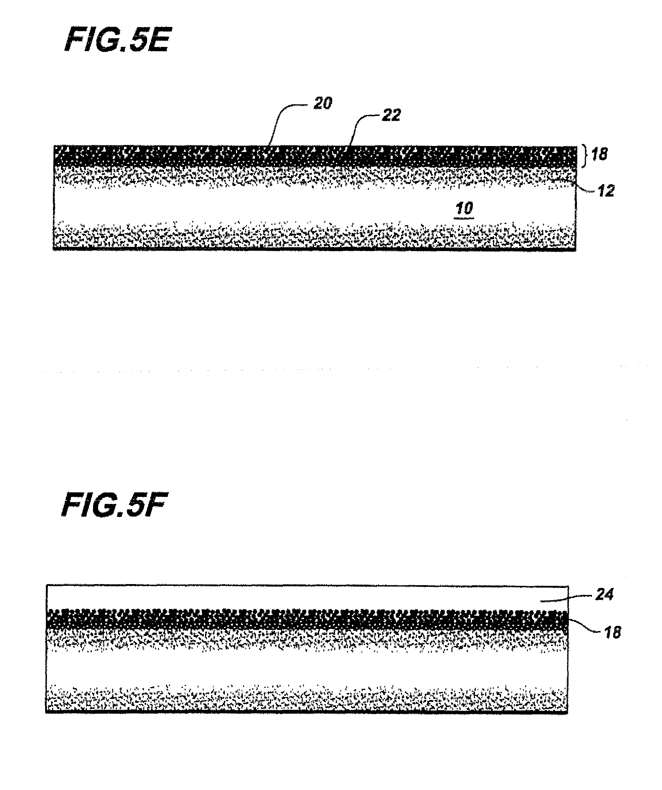

FIG. 5D illustrates the step of irradiating the first deposited drug layer 16 with GCIB gas clusters 18. This results in the formation of a first adhered drug layer 18, which is comprised of two primary components, such as shown in FIG. 5E. First adhered drug layer 18, and subsequently formed adhered drug layers, each include a carbonized drug matrix 20 having a plurality of interstices 22 in which will be disposed the remainder of the deposited drug that was not carbonized by the GCIB. Drug layer 18 is adhered to the surface region 12, and a portion of the non-carbonized drug will be released at an expected rate (characterized as an elution profile) from the adhered drug layer 18 by diffusion through the interstices 22 of the carbonized drug matrix 20. A number of the interstices 22 are interconnected, and a portion of the interstices are open at each surface of the drug matrix 20 so as to permit non-carbonized drug to eventually elute from a substantial number of the interstices 22 of the drug matrix 20.

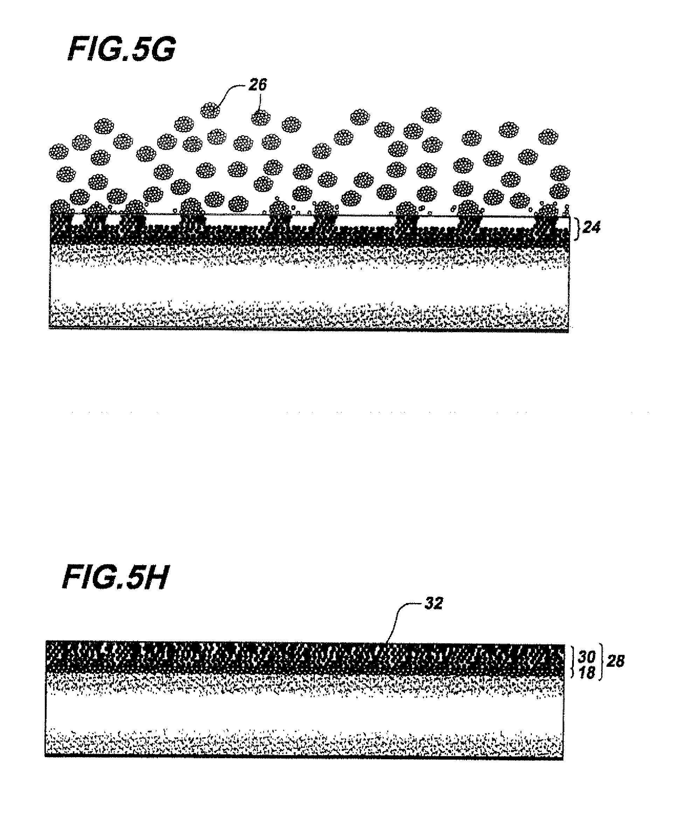

FIGS. 5F-5H illustrate how the drug deposition and GCIB irradiation process steps may be repeated, generally, to achieve multi-layered drug delivery structures having variable and extremely accurate drug loading. More particularly, FIG. 5F illustrates a second drug layer 24 deposited upon the first adhered drug layer 18 using the same or an alternative deposition process. The second drug layer 24 is then irradiated (FIG. 5G) with GCIB gas clusters 26 delivering substantially similar dosing or different, depending upon desired elution profile. Similar GCIB irradiation doses delivered to substantially similar or identical therapeutic agents will result in substantially similar elution profiles between or among adhered layers. FIG. 5H illustrates a drug delivery system comprised of an adhered drug layer 28 that is further comprised of the first adhered drug layer 18 and a second adhered drug layer 30. As many repetitions of the drug deposition and GCIB irradiation steps as needed to attain an overall elution profile, or profiles (if multiple therapeutic agents are utilized), may be performed. In one preferred embodiment, the first adhered drug layer 18 and second adhered drug layer 30 are similarly formed to have similar elution profiles, such that, as drug is released from the interstices 32 of layer 30, drug eluting from layer 18 into layer 30 replenishes the released drug. The adhered drug layers 18, 30 are not necessarily, however, comprised of the same drug substance(s).

Several alternative drug delivery systems in accordance with the present invention will now be described, with reference to FIGS. 6A-6B.

As noted above, multiple factors, including the thickness of the deposited drug layer, will determine whether GCIB gas clusters will penetrate a deposited drug layer so as to reach the surface onto which a new drug layer is to be adhered. FIG. 6A (and FIG. 5E) illustrates a drug delivery system 38 that is further comprised of spatially distinct adhered drug structures 34-36 formed when GCIB gas clusters penetrate a thinly deposited drug layer (*e*.*g*., on the order of several to tens of Angstroms, or greater.) Note that some portion of the adhered drug structures 34-36 are bonded (or stitched) to associated, spatially distinct surface regions 12-14. Formation of each of the adhered drug structures 34-36 may be accomplished nearly simultaneously or in separate processing routines. The therapeutic agent to be released from each of the adhered drug structures 34-36 is deposited at the associated spatially distinct surface region 12-14 and then GCIB irradiated. Again, the drug deposited at each surface region 12-14 is not necessarily the same. Forming adhered drug structures on less than the entire surface of the medical device has the benefit of cost savings when an expensive drug is to be used. Also, certain drugs may only need to be delivered at particular locations, such as at a site of significant tissue interaction with an implanted medical device.

FIG. 6B illustrates an alternative embodiment of a drug delivery system, such as may be formed when the GCIB does not penetrate the thickness of a drug layer deposited on the surface region 12 of the medical device 10. In such embodiments, a carbonized drug matrix 22 is still formed having interstices within which some portion of non-carbonized drug is disposed, and from which non-carbonized drug is released, however the drug matrix 22 does not extend to the surface 12 of the medical device 10. Rather, the carbonized matrix 22 encapsulates the remainder of deposited drug 24 that was not carbonized by the GCIB (and not captured in the interstices), between the drug matrix 22 and the surface 12 of the device 10. As noted above, the expression "adhered drug layer" as used herein refers collectively to the carbonized matrix 22, and the non-carbonized portions of the deposited drug, whether disposed in the interstices or encapsulated by the drug matrix 22 and the device surface.

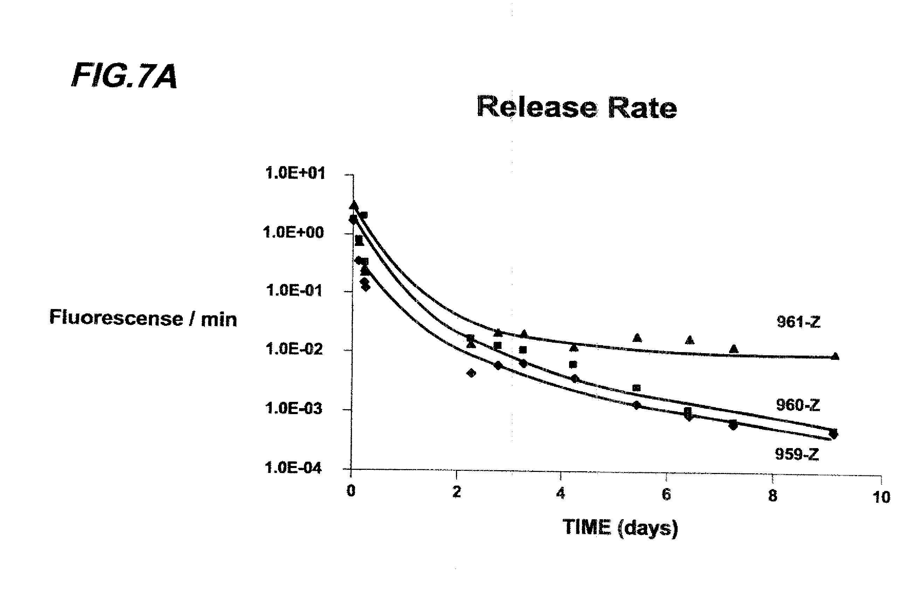

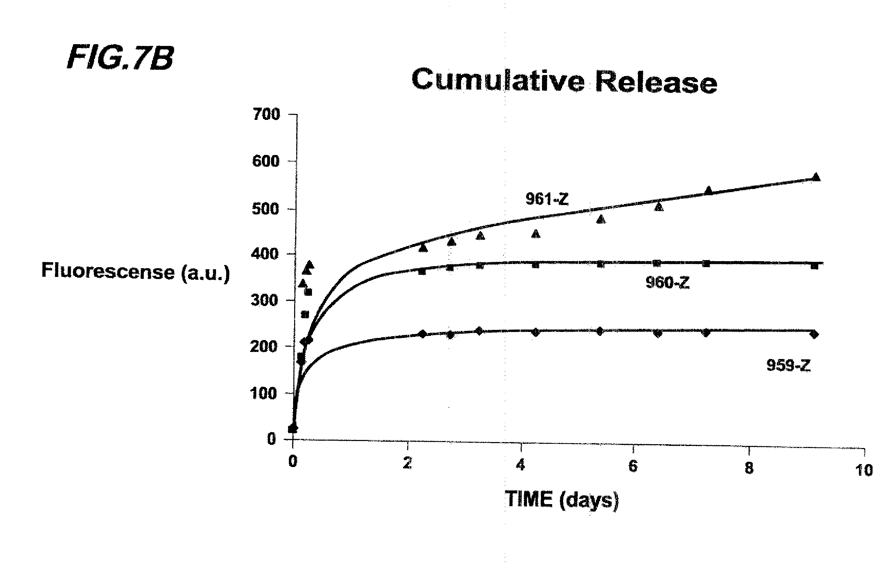

Now turning to FIGS. 7A and 7B, elution rates for a substance adhered to a surface of a coronary stent using GCIB processing in accordance with one embodiment of the present invention is shown. To demonstrate the release rate of a molecule adhered to the surface in accordance with the present invention, the surface was irradiated and a flourescent organic dye was vapor deposited onto the freshly irradiated surface while the surface remained in the vacuum chamber. The dye elution rate was measured by observing the flourescence of the elute as a function of time. In FIG. 7A, the release rate is shown over time. In FIG. 7B, the cumulative release rate is shown over time.

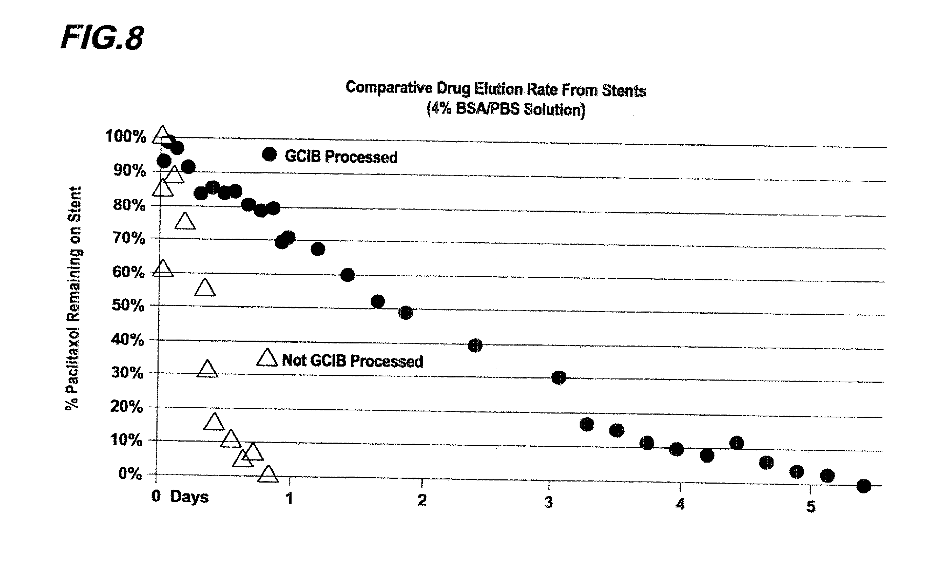

FIG. 8 illustrates results of comparative elution rate testing performed on a conventional drug-coated stent and a stent upon which an exemplary drug delivery system has been firmed utilizing GCIB irradiation in accordance with the present invention. Paclitaxol was selected as the test drug, which in the case of the non-GCIB processed stent was deposited, and for the GCIB processed stent was deposited by ultrasonic atomization prior to being irradiated with an Argon GCIB while rotating the stent between 3-5 RPM. The Paclitaxol was allowed to elute from the respective stents over time into a 4% Bovine Serum Albumin/Phosphate Buffered Saline solution, and the drug remaining on the stents was measured. As shown, significantly more drug remained loaded on the drug-adhered stent for a longer period of time that the conventional drug coated stent

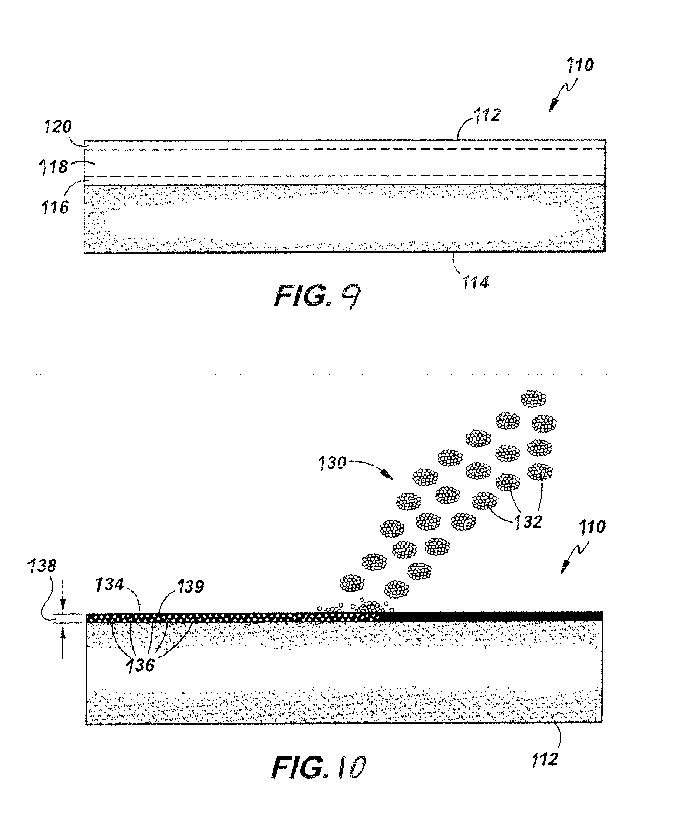

With reference to FIG. 9, a drug delivery system 110, which includes a drug containing medium 112 and an optional substrate or medical device 114, is shown prior to processing by the method of the present invention. Medical device 114 is only representational and may take any suitable form. Devise 114 may include an implantable medical device such as a stent or any other medical device which may benefit from an in situ drug delivery mechanism. Optionally, the use of substrate or device 114 may be limited to the fabrication of drug containing medium 112, wherein substrate or device 114 is removed from medium 112 prior to implantation. Substrate or device 114 maybe he constructed of any suitable material such as, for example, metal, ceramic or a polymer. Portions of substrate or device 114 may also be surface treated using GCIB in accordance with the method mentioned above, prior to the application of drug/polymer medium 112.

Drug containing medium 112 may take any suitable form such as the various polymer arrangements discussed above. Medium 112 may include just a single layer of drug containing material, or it may include multiple layers 116, 118, 120, as described above. Although the existing art identifies the use of an outer layer to control initial drug release, the process of the present invention may be used with this known arrangement to further control surface characteristics of the medium, including the drug release rate after initial in situ liquid exposure. Drug medium 112 may be applied to device 114 in any suitable arrangement from just a portion to complete or almost complete enclosure of device 114.

One method of application of medium 112 to device 114 uses a drug polymer mixture with a volatile solvent, which is deposited upon a surface of device 114. The solvent is evaporated to leave a cohesive drug/polymer mixture in the form of medium 112, attached to the substrate. Once the solvent is evaporated, drug medium 112 may form a cohesive mixture or mass and thereby provide a suitable drug delivery system, even in the absence of device 114.

With reference to FIG. 10, the drug delivery system 110 is shown undergoing irradiation with a gas cluster ion beam stream 130 of gas cluster molecules is being scanned across the cross section of drug delivery device 110. The clusters 132 break up upon impact with the surface 134 resulting in the shallow implantation of individual or small groups of molecules 136. Most of the individual molecules 136 stop within the first couple of molecular levels of medium 112 with the result that most of a thin layer 138 at surface 134 is densified or carbonized by the impinging molecules. The sealing of surface 134 is not complete, as various openings 139 remain in surface 134 which openings allow for the elution of drugs from medium 112. Thus, it is through the amount of GCIB irradiation that the characteristics of surface 134 are determined. The greater the amount of irradiation, the fewer and smaller are the openings in surface 134, thereby slowing the release of drugs from medium 112. Also, this densification or carbonization of surface 134 causes pacification or sealing of surface 134, which can decrease the bio-reactivity of surface 134 in contact with living tissue. In the case of some polymer materials which may be used for medium 112, the densification or carbonization can limit the release of volatile organic compounds by the medium 112 into surrounding living tissue. Thus, the process of the present invention enhances the choices of materials which may be used to construct medium 12 and can reduce risk factors associated with those material choices

Studies have suggested that a wide variety of drugs may be useful at the site of contact between the medical device and the in situ environment. For example, drugs such as anti-coagulants, anti-prolifics, antibiotics, immune-supressing agents, vasodilators, anti-thrombotic substances, anti-platelet substances, and cholesterol reducing agents may reduce instances of restenosis when diffused into the blood vessel wall after insertion of the stent, Although the present invention is described in reference to stents, its applications and the claims hereof are not limited to stents and may include any contact with a living body where drug delivery may be helpful

Although the invention has been described with respect to various embodiments, it should be realized this invention is also capable of a wide variety of further and other embodiments within the scope of the appended claims.

## Claims

1. Method of producing a drug delivery system, comprising the steps of:
(a) selecting a first drug substance;
(b) depositing the first drug substance onto at least one surface region of a medical device so as to form a first deposited drug layer;
(c) forming a first gas cluster ion beam in a vacuum chamber;
(d) positioning the at least one surface region of the medical device in the vacuum chamber for irradiation by the first gas cluster ion beam;
(e) irradiating the first deposited drug layer with the first gas cluster ion beam so as to adhere a drug layer to the at least one surface region of the medical device such that a portion of the deposited drug substance is permitted to be released from the first adhered drug layer at a first expected rate; wherein
the adhered drug layer comprises at least one carbonized matrix formed of a portion of the first deposited drug layer and including a plurality of interstices within which is disposed a non-carbonized portion of the first deposited drug layer, a plurality of the interstices being open at a surface of the carbonized matrix so as to permit the non-carbonized portion of the first deposited drug layer to be released at the expected rate; and wherein the at least one adhered drug layer further comprises another non-carbonized portion of the first deposited drug layer that is encapsulated between the carbonized matrix and the at least one surface region of the medical device;
(f) selecting an additional drug substance;
(g) depositing the additional drug substance as an additional drug layer onto the most recently adhered drug layer;
(h) irradiating the additional drug layer with an additional gas cluster ion beam so as to adhere the additional drug layer onto the most recently adhered drug layer such that a portion of the additional deposited drug substance is permitted to be released from the additional adhered drug layer at an additional expected rate; and
(i) optionally repeating steps (f) through (i) until a desired number of additional adhered drug layers are formed.

2. The method of claim 1, wherein the first gas cluster beam and the additional gas cluster ion beam(s) deliver at least two different irradiation doses resulting in at least two different drug elution profiles between/among the first adhered drug layer and the additional adhered drug layer(s).

3. The method of claim 1, wherein:
the at least one surface region comprises a plurality of spatially distinct regions of the surface of the medical device;
the first deposited drug layer is comprised of a corresponding plurality of drug substance portions; and
the depositing step comprises depositing each portion of the drug substance onto a corresponding one of the plurality of spatially distinct regions.

4. The method of claim 1, further comprising gas cluster ion beam irradiating the at least one surface region of the medical device prior to depositing the drug substance so as to smoothen the at least one surface region.

5. The method of claim 1, wherein the depositing step comprises any of:
vapor phase depositing the drug substance onto the at least one surface region; or
spraying the drug substance onto the at least one surface region; or ultrasonically atomizing the drug substance onto the at least one surface region; or
electrostatically coating the at least one surface region with a drug substance in powder form; or
sublimating the drug substance onto the at least one surface region.

6. The method of claim 1, wherein the medical device surface is comprised of at least one material selected from polymers, metals and ceramics.

7. The method of claim 1, wherein one or more of step (a) and/or step(s) (f) further comprise selecting the first and/or additional drug substance(s) from the group consisting of anti-coagulants, antibiotics, anti-tumor substances, immune-suppressing agents, vasodilators, anti-prolifics, anti-thrombotic substances, anti-platelet substances, cholesterol reducing agents and combinations thereof

8. A drug delivery system, comprising:
a medical device having at least one surface region;
a first drug layer adhered to the at least one surface region, the first drug layer comprised of at least one carbonized drug matrix of a first drug substance, the carbonized drug matrix including a plurality of interstices within which is disposed a non-carbonized portion of the first drug substance, a portion of the interstices being open at a surface of the carbonized drug matrix so as to permit the non-carbonized portion of the first drug substance to be released from the carbonized drug matrix at an expected rate; wherein the first adhered drug layer further comprises another non-carbonized portion of the first deposited drug layer encapsulated between the carbonized matrix and the at least one surface region of the medical device; and
at least one additional drug layer adhered to the first drug layer, the additional adhered drug layer comprised of at least one additional carbonized drug matrix of an additional drug substance, the at least one additional carbonized drug matrix including a plurality of interstices within which is disposed a non-carbonized portion of the additional drug substance, a portion of the interstices being open at a surface of the at least one additional carbonized drug matrix so as to permit the non-carbonized portion of the additional drug substance to be released from the at least one additional carbonized drug matrix at an expected rate and to permit the non-carbonized portion of the first drug substance to elute from the first adhered drug layer into the at least one additional adhered drug layer.

9. The drug delivery system of claim 8 wherein the first adhered drug layer and the at least one additional adhered drug layer have different drug elution profiles.

10. The drug delivery system of claim 8, wherein either of the first drug substance and/or the additional drug substance is of a type respectively selected from the group consisting of anti-coagulants, antibiotics, anti-tumor substances, immune-suppressing agents, vasodilators, anti-prolifics, anti-thrombotic substances, anti-platelet substances, cholesterol reducing agents and combinations thereof.

## Patentansprüche

1. Verfahren zum Erzeugen eines Arzneimittelzuführungssystems, das die folgenden Schritte beinhaltet:
(a) Auswählen einer ersten Arzneimittelsubstanz;
(b) Abscheiden der ersten Arzneimittelsubstanz auf wenigstens eine Oberflächenregion einer medizinischen Vorrichtung, um eine erste abgeschiedene Arzneimittelschicht zu bilden;
(c) Bilden eines ersten Gascluster-Ionenstrahls in einer Vakuumkammer;
(d) Positionieren der wenigstens einen Oberflächenregion der medizinischen Vorrichtung in der Vakuumkammer zum Bestrahlen mit dem ersten Gascluster-Ionenstrahl;
(e) Bestrahlen der ersten abgeschiedenen Arzneimittelschicht mit dem ersten Gascluster-Ionenstrahl, um eine Arzneimittelschicht auf die wenigstens eine Oberflächenregion der medizinischen Vorrichtung zu kleben, so dass ein Teil der abgeschiedenen Arzneimittelsubstanz von der ersten anhaftenden Arzneimittelschicht mit einer ersten erwarteten Rate freigesetzt werden kann; wobei
die anhaftende Arzneimittelschicht wenigstens eine carbonisierte Matrix beinhaltet, die aus einem Teil der ersten abgeschiedenen Arzneimittelschicht gebildet ist und mehrere Zwischenräume aufweist, innerhalb derer sich ein nicht carbonisierter Teil der ersten abgeschiedenen Arzneimittelschicht befindet, wobei mehrere der Zwischenräume an einer Oberfläche der carbonisierten Matrix offen sind, so dass der nicht carbonisierte Teil der ersten abgeschiedenen Arzneimittelschicht mit der erwarteten Rate freigesetzt werden kann; und wobei die wenigstens eine anhaftende Arzneimittelschicht ferner einen anderen nicht carbonisierten Teil der ersten abgeschiedenen Arzneimittelschicht beinhaltet, der zwischen der carbonisierten Matrix und der wenigstens einen Oberflächenregion der medizinischen Vorrichtung eingekapselt ist;
(f) Auswählen einer zusätzlichen Arzneimittelsubstanz;
(g) Abscheiden der zusätzlichen Arzneimittelsubstanz als zusätzliche Arzneimittelschicht auf die zuletzt aufgeklebte Arzneimittelschicht;
(h) Bestrahlen der zusätzlichen Arzneimittelschicht mit einem zusätzlichen Gascluster-Ionenstrahl, um die zusätzliche Arzneimittelschicht auf die zuletzt aufgeklebte Arzneimittelschicht zu kleben, so dass ein Teil der zusätzlichen abgeschiedenen Arzneimittelschicht von der zusätzlichen aufgeklebten Arzneimittelschicht mit einer zusätzlichen erwarteten Rate freigesetzt werden kann; und
(i) optional Wiederholen der Schritte (f) bis (i), bis eine gewünschte Anzahl von zusätzlichen anhaftenden Arzneimittelschichten gebildet ist.

2. Verfahren nach Anspruch 1, wobei der erste Gasclusterstrahl und der/die zusätzliche(n) Gascluster-Ionenstrahl(en) wenigstens zwei unterschiedliche Bestrahlungsdosen zuführt/zuführen, die in wenigstens zwei unterschiedlichen Arzneimittelelutionsprofilen zwischen der ersten anhaftenden Arzneimittelschicht und der/den zusätzlichen anhaftenden Arzneimittelschicht(en) resultieren.

3. Verfahren nach Anspruch 1, wobei:
die wenigstens eine Oberflächenregion mehrere räumlich getrennte Regionen der Oberfläche der medizinischen Vorrichtung aufweist;
die erste abgeschiedene Arzneimittelschicht aus einer entsprechenden Mehrzahl von Arzneimittelsubstanzteilen besteht; und
der Abscheidungsschritt das Abscheiden jedes Teils der Arzneimittelsubstanz auf eine entsprechende eine der mehreren räumlich getrennten Regionen beinhaltet.

4. Verfahren nach Anspruch 1, das ferner eine Bestrahlung der wenigstens einen Oberflächenregion der medizinischen Vorrichtung mit Gascluster-Ionenstrahlen vor dem Abscheiden der Arzneimittelsubstanz beinhaltet, um die wenigstens eine Oberflächenregion zu glätten.

5. Verfahren nach Anspruch 1, wobei der Abscheidungsschritt wenigstens eines der Folgenden beinhaltet:
Dampfphasenabscheiden der Arzneimittelsubstanz auf die wenigstens eine Oberflächenregion; oder
Sprühen der Arzneimittelsubstanz auf die wenigstens eine Oberflächenregion; oder Ultraschallzerstäuben der Arzneimittelsubstanz auf die wenigstens eine Oberflächenregion; oder
elektrostatisches Beschichten der wenigstens einen Oberflächenregion mit einer Arzneimittelsubstanz in Pulverform; oder
Sublimieren der Arzneimittelsubstanz auf die wenigstens eine Oberflächenregion.

6. Verfahren nach Anspruch 1, wobei die Oberfläche der medizinischen Vorrichtung aus wenigstens einem Material besteht, das aus Polymeren, Metallen und Keramik ausgewählt ist.

7. Verfahren nach Anspruch 1, wobei einer oder mehrere von Schritt (a) und/oder Schritt(en) (f) ferner das Auswählen der ersten und/oder zusätzlichen Arzneimittelsubstanz(en) aus der Gruppe bestehend aus Antikoagulationsmitteln, Antibiotika, Anti-Tumor-Substanzen, Immununterdrückungsmitteln, Vasodilatatoren, anti-proliferativen Mitteln, antithrombotischen Substanzen, Anti-Plättchensubstanzen, Cholesterin reduzierenden Mitteln und Kombinationen davon beinhaltet/beinhalten.

8. Arzneimittelzuführungssystem, das Folgendes umfasst:
eine medizinische Vorrichtung mit wenigstens einer Oberflächenregion;
eine erste Arzneimittelschicht, die an der wenigstens einen Oberflächenregion haftet, wobei die erste Arzneimittelschicht aus wenigstens einer carbonisierten Arzneimittelmatrix einer ersten Arzneimittelsubstanz besteht, wobei die carbonisierte Arzneimittelmatrix mehrere Zwischenräume aufweist, in denen ein nicht carbonisierter Teil der ersten Arzneimittelsubstanz angeordnet ist, wobei ein Teil der Zwischenräume an einer Oberfläche der carbonisierten Arzneimittelmatrix offen ist, so dass der nicht carbonisierte Teil der ersten Arzneimittelsubstanz von der carbonisierten Arzneimittelmatrix mit einer erwarteten Rate freigesetzt werden kann; wobei die erste anhaftende Arzneimittelschicht ferner einen anderen nicht carbonisierten Teil der ersten abgeschiedenen Arzneimittelschicht beinhaltet, der zwischen der carbonisierten Matrix und der wenigstens einen Oberflächenregion der medizinischen Vorrichtung eingekapselt ist; und
wenigstens eine zusätzliche Arzneimittelschicht, die an der ersten Arzneimittelschicht haftet, wobei die zusätzliche anhaftende Arzneimittelschicht aus wenigstens einer zusätzlichen carbonisierten Arzneimittelmatrix einer zusätzlichen Arzneimittelsubstanz besteht, wobei die wenigstens eine zusätzliche carbonisierte Arzneimittelmatrix mehrere Zwischenräume beinhaltet, in denen ein nicht carbonisierter Teil der zusätzlichen Arzneimittelmatrix angeordnet ist, wobei ein Teil der Zwischenräume an einer Oberfläche der wenigstens einen zusätzlichen carbonisierten Arzneimittelmatrix offen ist, so dass der nicht carbonisierte Teil der zusätzlichen Arzneimittelsubstanz von der wenigstens einen zusätzlichen carbonisierten Arzneimittelmatrix mit einer erwarteten Rate freigesetzt werden kann und der nicht carbonisierte Teil der ersten Arzneimittelsubstanz von der ersten anhaftenden Arzneimittelschicht in die wenigstens eine zusätzliche anhaftende Arzneimittelschicht eluiert werden kann.

9. Arzneimittelzuführungssystem nach Anspruch 8, wobei die erste anhaftende Arzneimittelschicht und die wenigstens eine zusätzliche anhaftende Arzneimittelschicht unterschiedliche Arzneimittelelutionsprofile haben.

10. Arzneimittelzuführungssystem nach Anspruch 8, wobei entweder die erste Arzneimittelsubstanz und/oder die zusätzliche Arzneimittelsubstanz von einem Typ ist/sind, der aus der Gruppe bestehend aus Antikoagulationsmitteln, Antibiotika, Anti-Tumor-Substanzen, immununterdrückenden Mitteln, Vasodilatatoren, anti-proliferativen Mitteln, antithrombotischen Substanzen, Anti-Plättchensubstanzen, Cholesterin reduzierenden Mitteln und Kombinationen davon ausgewählt ist.

## Revendications

1. Procédé de production d'un système d'administration d'un médicament, qui comprend les étapes consistant à :
(a) sélectionner une première substance médicamenteuse ;
(b) déposer la première substance médicamenteuse sur au moins une région en surface d'un dispositif médical de manière à former une première couche médicamenteuse déposée ;
(c) former un premier faisceau ionique d'agrégats gazeux dans une chambre à vide ;
(d) placer la au moins une région en surface du dispositif médical dans la chambre à vide pour l'irradier avec le premier faisceau ionique d'agrégats gazeux ;
(e) irradier la première couche médicamenteuse déposée avec le premier faisceau ionique d'agrégats gazeux de manière à faire adhérer une couche médicamenteuse sur la au moins une région en surface du dispositif médical afin qu'une portion de la substance médicamenteuse déposée puisse être libérée de la première couche médicamenteuse adhérée à un premier taux attendu ;
où la couche médicamenteuse adhérée comprend au moins une matrice carbonisée qui est formée d'une portion de la première couche médicamenteuse déposée et qui inclut une pluralité d'interstices dans lesquels est située une portion non carbonisée de la première couche médicamenteuse déposée, une pluralité des interstices étant ouverts à une surface de la matrice carbonisée pour permettre la libération de la portion non carbonisée de la première couche médicamenteuse déposée au taux attendu ; et où la au moins une couche médicamenteuse adhérée comprend en outre une autre portion non carbonisée de la première couche médicamenteuse déposée qui est encapsulée entre la matrice carbonisée et la au moins une région en surface du dispositif médical ;
(f) sélectionner une substance médicamenteuse supplémentaire ;
(g) déposer la substance médicamenteuse supplémentaire sous la forme d'une couche médicamenteuse supplémentaire sur la couche médicamenteuse la plus récemment adhérée ;
(h) irradier la couche médicamenteuse supplémentaire avec un faisceau ionique d'agrégats gazeux supplémentaire de manière à faire adhérer la couche médicamenteuse supplémentaire sur la couche médicamenteuse la plus récemment adhérée afin qu'une portion de la substance médicamenteuse supplémentaire déposée puisse être libérée de la couche médicamenteuse adhérée supplémentaire à un autre taux attendu ; et
(i) en option répéter les étapes (f) à (i) jusqu'à ce qu' un nombre désiré de couches médicamenteuses adhérées supplémentaires ait été formé.

2. Procédé de la revendication 1, dans lequel le premier faisceau d'agrégats gazeux et le(s) faisceau(x) ionique(s) d'agrégats gazeux supplémentaire(s) délivrent au moins deux doses d'irradiation différentes résultant en au moins deux profils d'élution médicamenteuse différents entre/parmi la première couche médicamenteuse adhérée et la ou les couche(s) médicamenteuse(s) adhérée(s) supplémentaire(s).

3. Procédé de la revendication 1, dans lequel :
la au moins une région en surface comprend une pluralité des zones spatialement distinctes de la surface du dispositif médical ;
la première couche médicamenteuse déposée est composée d'une pluralité correspondante de portions de la substance médicamenteuse ; et
l'étape de dépôt comprend le dépôt de chaque portion de la substance médicamenteuse sur une zone correspondante de la pluralité de zones spatialement distinctes.

4. Procédé de la revendication 1, qui comprend en outre l'irradiation, par le faisceau ionique d'agrégats gazeux, de la au moins une région en surface du dispositif médical avant le dépôt de la substance médicamenteuse afin d'aplanir la au moins une région en surface.

5. Procédé de la revendication 1, dans lequel l'étape de dépôt à l'une quelconque des techniques suivantes :
dépôt de la substance médicamenteuse en phase vapeur sur la au moins une région en surface ; ou
pulvérisation de la substance médicamenteuse sur la au moins une région en surface ; ou
atomisation ultrasonique de la substance médicamenteuse sur la au moins une région en surface ; ou
revêtement électrostatique de la au moins une région en surface avec une substance médicamenteuse sous la forme d'une poudre ; ou
sublimation de la substance médicamenteuse sur la au moins une région en surface.

6. Procédé de la revendication 1, dans lequel la surface du dispositif médical est composée d'au moins un matériau sélectionné parmi des polymères, des métaux et des céramiques.

7. Procédé de la revendication 1, dans l'étape (a) et/ou l'étape ou les étapes (f) comprennent en outre la sélection de la première substance médicamenteuse et/ou d'une ou de plusieurs substances médicamenteuses supplémentaires sélectionnées dans le groupe consistant en les suivantes : anticoagulants, antibiotiques, antitumoraux, immunosuppresseurs, vasodilatateurs, antiproliférateurs, antithrombiques, antiagrégants plaquettaires, hypocholestérolémiants et combinaisons de ceux-ci.

8. Système d'administration d'un médicament qui comprend :
un dispositif médical ayant au moins une région en surface ;
une première couche médicamenteuse adhérant à la au moins une région en surface, où la première couche médicamenteuse comprend au moins une matrice médicamenteuse carbonisée d'une première substance médicamenteuse, où la matrice médicamenteuse carbonisée inclut une pluralité d'interstices dans lesquels est située une portion non carbonisée de la première substance médicamenteuse et où une portion des interstices est ouverte à une surface de la matrice médicamenteuse carbonisée pour permettre la libération de la portion non carbonisée de la première substance médicamenteuse à partir de la matrice médicamenteuse carbonisée à un taux attendu ; et où la première couche médicamenteuse adhérée comprend une autre portion non carbonisée de la première couche médicamenteuse déposée encapsulée entre la matrice carbonisée et la au moins une région en surface du dispositif médical ; et
au moins une couche médicamenteuse supplémentaire adhérant à la première couche médicamenteuse, où la couche médicamenteuse adhérée supplémentaire comprend au moins une matrice médicamenteuse carbonisée supplémentaire d'une substance médicamenteuse supplémentaire, où la une au moins matrice médicamenteuse carbonisée supplémentaire inclut une pluralité d'interstices dans lesquels est située une portion non carbonisée de la substance médicamenteuse supplémentaire et où une portion des interstices est ouverte à une surface de la au moins matrice médicamenteuse carbonisée supplémentaire pour permettre la libération de la portion non carbonisée de la substance médicamenteuse supplémentaire à partir de la au moins une matrice médicamenteuse carbonisée supplémentaire à un taux attendu et pour permettre l'élution de la portion non carbonisée de la première substance médicamenteuse de la première couche médicamenteuse adhérée vers la au moins une couche médicamenteuse adhérée supplémentaire.

9. Système d'administration d'un médicament de la revendication 8, dans lequel la première couche médicamenteuse adhérée et la au moins une couche médicamenteuse adhérée supplémentaire ont des profils d'élution médicamenteuse différents.

10. Système d'administration d'un médicament de la revendication 8, dans lequel la première substance médicamenteuse et/ou la substance médicamenteuse supplémentaire sont respectivement d'un type sélectionné dans le groupe consistant en les suivants : anticoagulants, antibiotiques, antitumoraux, immunosuppresseurs, vasodilatateurs, antiproliférateurs, antithrombiques, antiagrégants plaquettaires, hypocholestérolémiants et combinaisons de ceux-ci.
